# EUROPEAN PATENT APPLICATION

(11) **EP 3 978 008 A1**
(43) Date of publication of application: **06.04.2022**
(21) Application number: 20199971.1
(22) Date of filing: 03.10.2020
(51) Int. Cl.: A61K 36/71, A61K 8/19, A61K 41/00, A61K 47/69, A61P 31/14

(54) **COMPOSITIONS WITH NIGELLA SATIVA EXTRACTS**

(71) Applicant: Fetian, Issa Rasheed, 4132 Muttenz (CH)
(72) Inventor: Fetian, Issa Rasheed, 4132 Muttenz (CH)
(74) Representative: Gernet Althaus IP AG

(57) **Abstract**

The invention relates to compositions comprising an extract of Nigella sativa for use in the treatment of a viral infection with SARS-CoV-2.

## Description

The present invention relates to compositions comprising Nigella sativa extracts and their use in the treatment of viral infections with Severe Acute Respiratory Syndrome Coronavirus 2 (SARS-CoV-2).

Coronavirus Disease 2019 (COVID-19) has been widely spreading all over the world since the beginning of 2020. Although severe transient restrictions in economic and personal life helped to bring about a slowdown in the spreading of the disease, infections with SARS-CoV-2 are still quite frequently occurring and the number of infections is again rising in most countries. World Health Organization, on January 30, 2020, declared the spread of COVID-19 to be a public emergency of international concern. As of September 30, 2020, 33'692'221 cases and 1'008'842 deaths due to COVID-19 have been reported. The disease is transmitted via inhalation or contact with virus-containing droplets. The incubation period ranges from two to fourteen days or even longer. The estimated fatality rate ranges from two to three percent. The virus can be detected in respiratory secretions by special molecular tests, such as polymerase chain reaction (PCR) based tests detecting specific sequences in the RNA genome of SARS-CoV-2. Elevated levels of C-reactive protein have been detected in the blood samples of patients, while the white blood cell counts were considered normal. The computerized tomographic chest scan is usually abnormal even in those subjects with no symptoms or mild disease.

Current treatment options are essentially supportive, as the role of antiviral agents is yet to be established. Preventative measures can be utilized to slow the spread of infection, such as isolation of suspected cases or those with mild symptoms and strict infection control measures at hospitals.

Though the SARS-CoV-2 is far more infectious than its two ancestors, Severe acute respiratory syndrome coronavirus (SARS-CoV) and Middle East respiratory syndrome coronavirus (MERS-CoV), SARS-CoV-2 has a lower mortality rate. To date, the impact of this pandemic is uncertain, and the global research community is working diligently to find a satisfactory therapy.

Different agents, e.g. chloroquine and Remdesivir, the latter being an antiviral compound that had been developed to fight Ebola virus infections, have been or are tested on the suitability to treat SARS-CoV-2 infections. However, the successful outcome of such treatments is yet unclear and remains to be elucidated in further studies. For other agents such as chloroquine, the side effects proved to be too severe so its use in the treatment of SARS-CoV-2 infections has largely been given up.

For thousands of years, plants have been a source of medicinal agents. Many of the drugs that are used today still come from plants, for example, morphine (opium poppy), digoxin (foxglove), and atropine (datura) to mention a few.

US 6 841 174 describes complex herbal compositions comprising herbal extracts of numerous plants. The composition is prepared in powder form using dried herbs. The herbal compositions are used for treating viral infections with hepatitis B and / or hepatitis C. The herbal compositions are administered to subjects in need thereof.

Currently, there are quite a number of treatment options for viral infections with SARS-CoV-2 available, be it for milder and also more severe courses of the disease, these treatment options are largely of supportive nature and there is up to date no satisfying therapy available. Thus, there is an ongoing need in the art for further options for treating viral infections with SARS-CoV-2. Moreover, there is a need in the art for treatment options that provide minimal or no side effects.

Accordingly, it is an object of the present invention to provide plant-based compositions for use in the treatment of viral infections with SARS-CoV-2. It is a further object of the present invention to provide plant-based compositions for use in the treatment of viral infections with SARS-CoV-2 that provide minimal side effects to the subject.

The object is achieved with a composition as defined in claim 1. Further, preferred embodiments are subject to dependent claims.

A composition according to the present invention comprises an extract of Nigella sativa. The composition is for use in the treatment of a viral infection with SARS-CoV-2.

Oil extraction may be done by press-extraction of Nigella sativa seeds and can be carried out using screw-less cold press (e.g. IBG Monforts Oekotec GmbH, Monchengladbach, Germany). Usually, the press-extraction is a cold-press procedure to utmost preserve the components of Nigella sativa oil.

Preparations of aqueous and ethanolic extracts of Nigella sativa may be made as follows: Nigella sativa seeds were commercially obtained. The seeds were cleaned and dried and powdered using a mechanical grinder. For the aqueous extract, the seed powder (100 g) was added to 1000 mL hotwater, boiled for 15 minutes, and filtered through a cloth. The filtrate was evaporated to dryness under reduced pressure to obtain a viscous residue. The residue was suspended in normal saline solution. For the ethanolic extract, the seed powders (100 g) were defatted with petroleum ether (40° to 60°C) using a Soxhlet apparatus known in the art. Then, the powder was macerated in 800 mL ethanol (80%, v/v) for 72 hours, and the mixture was subsequently filtered and concentrated in vacuo at 40°C. The residue was suspended in saline solution.

The composition comprising the Nigella sativa extract may further comprise a pharmaceutically-acceptable carrier. The composition may also be encapsulated in pharmaceutically-acceptable capsules. Such capsules are known in the art and are commercially available.

It has surprisingly been found that the composition according to the present invention is very effective for use in the treatment of viral infections with SARS-CoV-2. The various symptoms of a SARS-CoV-2 such as fever, (dry) cough, and shortness of breath are significantly alleviated within a few, typically about three days, from the beginning of the treatment. In some cases, a second test on virus particles of SARS-CoV-2, performed twelve days after the first test, yielded a negative result. Further, C-reactive protein (CRP), a clinical parameter, which has been found to undergo significant changes in subject having a severe course of the Corona virus disease 19 (COVID-19) caused by SARS-CoV-2, showed a statistically significant decrease after a few days of treatment with compositions according to the present invention. In addition, almost no side-effects were reported due to the intake of the composition according to the present invention. Thus, the side-effects of compositions according to the present invention for use in the treatment of a viral SARS-CoV-2 infection are minimal.

In a preferred embodiment the extract of Nigella sativa is further purified and comprises at least one active compound of Nigella sativa. The at least one active compound of Nigella sativa is selected from the group consisting of thymoquinone, thymohydroquinone, p-cymene, α-hederin, carvacrol, anethol, 4-terpineol, thymol, alpha-pinene, limonene, nigellidine, and sesquiterpene longifolene. The composition may also comprise a mixture of two, three or more active compounds of Nigella sativa.

The compound thymoquinone of Nigella sativa is a preferred active compound. Thymoquinone is a phytochemical compound found in the plant Nigella sativa which can be steam distilled to produce an essential oil. In laboratory experiments in cells and in animals, it has shown anti-inflammatory and antioxidant effects, and it has been studied in models of cardiovascular diseases and diabetes, neurodegenerative diseases and stroke, and cancer.

p-Cymene is a naturally occurring aromatic organic compound. p-Cymene is insoluble in water, but miscible with organic solvents. It is a constituent of a number of essential oils, most commonly the oil of cumin and thyme.

α-Hederin (alpha-hederin) is a water-soluble pentacyclic triterpenoid saponin found in the seeds of Nigella sativa. α-Hederin has been shown to enhance the cytotoxicity of an established chemotherapeutic agent, 5-fluorouracil, in an animal model of colon carcinoma.

Anethole is an organic compound that is widely used as a flavoring substance. It is a derivative of phenylpropene, a type of aromatic compound that occurs widely in nature, in essential oils.

Thymol (also known as 2-isopropyl-5-methylphenol, IPMP) is a natural monoterpenoid phenol derivative of cymene, C₁₀H₁₄O, isomeric with carvacrol, found in oil of thyme, and extracted from Thymus vulgaris (common thyme), and various other kinds of plants as a white crystalline substance of a pleasant aromatic odor and strong antiseptic properties. Thymol also provides the distinctive, strong flavor of the culinary herb thyme, also produced from T. vulgaris.

α-Pinene is an organic compound of the terpene class, one of two isomers of pinene. It is an alkene and it contains a reactive four-membered ring. It is found in the oils of many species of many coniferous trees, notably the pine. It is also found in the essential oil of rosemary (Rosmarinus officinalis) and Satureja myrtifolia.

Limonene is a colorless liquid aliphatic hydrocarbon classified as a cyclic monoterpene, and is the major component in the oil of citrus fruit peels. The D-isomer, occurring more commonly in nature as the fragrance of oranges, is a flavoring agent in food manufacturing. It is also used in chemical synthesis as a precursor to carvone and as a renewables-based solvent in cleaning products. The less common L-isomer is found in mint oils and has a piny, turpentine-like odor. The compound is one of the main volatile monoterpenes found in the resin of conifers, particularly in the Pinaceae, and of orange oil.

In table 1 the most prominent active compounds of Nigella sativa are shown:

**Table 1**

| | | | |
|---|---|---|---|
| Chemical Formula: | C10H12O2 | Chemical Formula: | C10H14O2 |
| Exact Mass: | 164,08 | Exact Mass: | 166,10 |
| Molecular Weight: | 164,20 | Molecular Weight: | 166,22 |
| m/z: | 164.08 (100.0%), 165.09 (10.8%) | m/z: | 166.10 (100.0%), 167.10 (10.8%) |
| Elemental Analysis: | C, 73.15; H, 7.37; O, 19.49 | Elemental Analysis: | C, 72.26; H, 8.49; O, 19.25 |
| 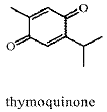 | | 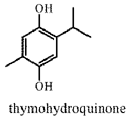 | |
| Chemical Formula: | C10H14 | Chemical Formula: | C41H66O12 |
| Exact Mass: | 134,11 | Exact Mass: | 750,46 |
| Molecular Weight: | 134,22 | Molecular Weight: | 750,97 |
| m/z: | 134.11 (100.0%), 135.11 (10.8%) | m/z: | 750.46 (100.0%), 751.46 (44.3%) 752.46 (9.6%), 752.46 (2.5%), 753.46 (1.1%) |
| Elemental Analysis: | C, 89.49; H, 10.51 | Elemental Analysis: | C, 65.58; H, 8.86; O, 25.57 |
| 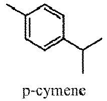 | | 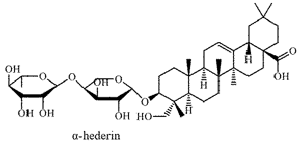 | |
| Chemical Formula: | C10H14O | Chemical Formula: | C10H12O |
| Exact Mass: | 150,10 | Exact Mass: | 148,09 |
| Molecular Weight: | 150,22 | Molecular Weight: | 148,21 |
| m/z: | 150.10 (100.0%), 151.11 (10.8%) | m/z: | 148.09 (100.0%), 149.09 (10.8%) |
| Elemental Analysis: | C, 79.96; H, 9.39; O, 10.65 | Elemental Analysis: | C, 81.04; H, 8.16; O, 10.80 |
| 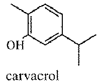 | | 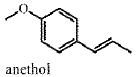 | |
| Chemical Formula: | C10H18O | Chemical Formula: | C10H14O |
| Exact Mass: | 154,14 | Exact Mass: | 150,10 |
| Molecular Weight: | 154,25 | Molecular Weight: | 150,22 |
| m/z: | 154.14 (100.0%), 155.14 (10.8%) | m/z: | 150.10 (100.0%), 151.11 (10.8%) |
| Elemental Analysis: | C, 77.87; H, 11.76; O, 10.37 | Elemental Analysis: | C, 79.96; H, 9.39; O, 10.65 |
| 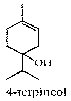 | | 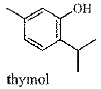 | |
| Chemical Formula: | C10H16 | Chemical Formula: | C10H16 |
| Exact Mass: | 136,13 | Exact Mass: | 136,13 |
| Molecular Weight: | 136,24 | Molecular Weight: | 136,24 |
| m/z: | 136.13 (100.0%), 137.13 (10.8%) | m/z: | 136.13 (100.0%), 137.13 (10.8%) |
| Elemental Analysis: | C, 88.16; H, 11.84 | Elemental Analysis: | C, 88.16; H, 11.84 |
| 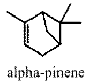 | | 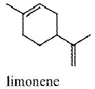 | |
| Chemical Formula: | C18H18N2O2 | Chemical Formula: | C10H14O |
| Exact Mass: | 294,14 | ExactMass: | 150,10 |
| Molecular Weight: | 294,35 | Molecular Weight: | 150,22 |
| m/z: | 294.14 (100.0%), 295.14 (19.5%), 296.14 (1.8%) | m/z: | 150.10 (100.0%), 151.11 (10.8%) |
| Elemental Analysis: | C, 73.45; H, 6.16; N, 9.52; O, 10.87 | Elemental Analysis: | C, 79.96; H, 9.39; O, 10.65 |
| 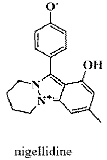 | | 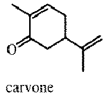 | |

In another preferred embodiment, the at least one active compound of the further purified extract of Nigella sativa is thymoquinone. Optionally, thymoquinone may be combined with one or more further active compounds of Nigella sativa. These further compounds are selected from the group consisting of thymohydroquinone, p-cymene, α-hederin, carvacrol, anethol, 4-terpineol, thymol, alpha-pinene, limonene, nigellidine, and sesquiterpene longifolene.

In yet another embodiment the composition comprising the extract of Nigella sativa for use in the treatment of a viral infection with SARS-CoV-2 further comprises tocopherol.

Tocopherols are a class of organic chemical compounds (more precisely, various methylated phenols), many of which have vitamin E activity. Thus, tocopherols are also referred to as vitamin E. Vitamin E exists in eight different forms, four tocopherols and four tocotrienols. All feature a chromane ring, with a hydroxyl group that can donate a hydrogen atom to reduce free radicals and a hydrophobic side chain that allows for penetration into biological membranes. Both the tocopherols and tocotrienols occur in a (alpha), β (beta), γ (gamma), and δ (delta) forms, determined by the number and position of methyl groups on the chromanol ring. Alpha-tocopherol is the form of vitamin E that is preferentially absorbed and accumulated in humans. The measurement of "vitamin E" activity in international units (IU) was based on fertility enhancement by the prevention of miscarriages in pregnant rats relative to α-tocopherol. Tocotrienols, although less commonly known, also belong to the vitamin E family.

The preferred tocopherol in compositions according to the present invention is α-tocopherol.

In another preferred embodiment the composition according to the present invention for use in the treatment of a viral infection with SARS-CoV-2 further comprises an iron oxide compound Iron oxides are chemical compounds composed of iron and oxygen. The iron oxide compound has oxidation state of (II) or (III), examples are Fe₂O₃ and Fe₃O₄. There is a number of known iron oxides, the best known of which is rust, a form of iron (III) oxide. Iron oxides and oxyhydroxides are widespread in nature and play an important role in many geological and biological processes. They are used as iron ores, pigments, catalysts, and in thermite, and occur in hemoglobin.

Kit of parts that comprises the composition for use in the treatment of a viral infection with SARS-CoV-2 according to the present invention and composition comprising ferrite nanoparticles. Preferably, the ferrite nanoparticles are solubilized or suspended in a solution suitable for intravenous injection.

In a preferred embodiment the ferrite nanoparticles are selected from the group consisting of magnetite (Fe₃O₄) and maghemite (Fe₂O₃).

The ferrite nanoparticles may be used in further method of treatment termed magnetic hyperthermia. Magnetic hyperthermia or biologically targeted magnetic hyperthermia may be used as a adjuvant treatment in subjects with a viral infection with SARS-CoV-2. Combined with compositions comprising an extract of Nigella sativa for use in the treatment of viral infections with SARS-CoV-2, magnetic hyperthermia or biologically targeted magnetic hyperthermia amplifies the effect of the Nigella sativa comprising composition. Preferably, the ferrite nanoparticles are biologically targeted such that they accumulate in the desired cells, tissue or biological structure. Targeting may for instance be achieved by way of an appropriate surface coating of the ferrite nanoparticles, e.g. such coating may comprise antibodies, parts of antibodies or other ligands the preferentially bind to the desired target cells or biological structure. After accumulation of the targeted ferrite nanoparticles an application of an alternating magnetic field follows to raise the temperature in the target cells or the target tissue. Such hyperthermia, the mild elevation of temperature (to 40-43°C) of cells or tissue in a living organism, can induce cell death and enhance the effects of another treatment, for example an antiviral treatment, by enhancing its cytotoxic effects.

The healthcare workers are facing challenges in reducing the severity and mortality of COVID-19 across the world. Severe patients with COVID-19 are generally treated in the intensive care unit, while mild or non-severe patients treated symptomatically at their own homes. However, there is an emerging challenge that a small subset of mild or non-severe COVID-19 patients develops into a severe disease course. Therefore, it is important to early identify and give the treatment of this subset of patients to reduce the disease severity and improve the outcomes of COVID-19. Clinical studies demonstrated that altered levels of some blood markers might be linked with the degree of severity and mortality of patients with COVID-19. Of these clinical parameters, serum C-reactive protein (CRP) has been found as an important marker that changes significantly in severe patients with COVID-19. CRP is a type of protein produced by the liver that serves as an early marker of infection and inflammation. In blood, the normal concentration of CRP is less than 10 mg/L; however, it rises rapidly within 6 to 8 hours and gives the highest peak in 48 hours from the disease onset. Its half-life is about 19 hours and its concentration decrease when the inflammatory stages end and the patient is healing. CRP preferably binds to phosphocholine expressed highly on the surface of damaged cells. This binding makes active the classical complement pathway of the immune system and modulates the phagocytic activity to clear microbes and damaged cells from the organism. When the inflammation or tissue damage is resolved, CRP concentration falls, making it a useful marker for monitoring disease severity.

The elevated levels of CRP might be linked to the overproduction of inflammatory cytokines in severe patients with COVID-19 or severe upper respiratory tract infection. Cytokines fight against the microbes but when the immune system becomes hyperactive, it can damage lung tissue. Thus, CRP production is induced by inflammatory cytokines and by tissue destruction in patients with COVID-19. Elevated level of CRP may be a valuable early marker in predicting the possibility of disease progression in non-severe patients with COVID-19, which can help health workers to identify those patients an early stage for early treatment. Besides, COVID-19 patients with elevated levels of CRP need close monitoring and treatment even though they did not develop symptoms to meet the criteria for the severe disease course.

Since the onset of the outbreak, many agents that could have efficacy against COVID-19 have been proposed. Various antiviral agents were included in the latest guidelines from the National Health Commission, including interferon, lopinavir/ritonavir, chloroquine phosphate, ribavirin and arbidol. Angiotensin receptor blockers, such as losartan, have also been suggested for the treatment of COVID-19.

As the number of Switzerland COVID-19 cases expands, many other healthcare providers are rolling out drive-through testing to help protecting patients and healthcare workers from contracting the virus.

A number of pharmaceutical and biological properties have been ascribed to the *N. sativa* such as antifungal, antibacterial, antiparasitic, and antiviral activity. Thymoquinon has shown efficacy against a variety of diseases, such as neurological and mental illnesses, cardiovascular disorders, cancer, diabetes, inflammatory conditions, and infertility. Nigella sativa has also recently gained increasing attention due to its strong antioxidant properties, leading to its frequent use as a dietary supplement. Additionally, thymoquinone has demonstrated synergistic effects with various chemotherapeutic agents, optimizing efficacy and reducing toxicity.

A routine SARS-CoV-2 PCR (nasopharyngeal swab, SARS-CoV-2 Test) was initially performed. According to the history of presenting illness of patients, the decision was taken to measure the infect parameters to determine the severity of the infection. As the result of the swab test in many hospitals or/and Institutes comes one to two days of testing date. A treatment decision was taken so that general condition of patients does not get worse, due to lack of possibilities for clinical examination of suspected cases in the light of pandemic disease and based on the infect parameter values as well the history of presenting illness

As the treatment options are limited and many therapies cannot be given to patients before the diagnostic process is finished, to many patients compositions comprising Nigella sativa extracts were given due to its strengthening effect.

Table 2 shows the detailed information and the outcome in those patients with suspected or confirmed COVID-19

**Table 2.**

| Subject | Clinical Features | Gender | Age | CRP mg/L | PCR SARS-CoV | Outcome |
|---|---|---|---|---|---|---|
| 1 | Patient presents to us with fever and sore throat | F | 23 | 38.4 | Negative | A clinically significant change in symptoms and CRP (within normal) was observed |
| 2 | Fever, dry cough, rhinitis symptoms, shortness of breath | M | 61 | within normal 0-10 | Positive | The shortness of breath disappeared after 3^{rd} day. Fever disappeared after two days of the therapy. |
| 3 | Fever and general weakness without any infect focus or flu symptoms | M | 73 | 123.4 | Negative | Fever was disappeared, and CRP was lower than 40 mg/L after 3 days of treatment |
| 4 | Dry cough, fever, and headache | F | 37 | 51.7 | Negative | The patient was recommended to have an appointment after the therapy but due to gain her health back the control appointment was canceled and the feed from patient was received by telephone |
| 5 | A 77-year-old female with a history of hypertension and hyperlipidemia who presented with fever, dry cough, malaise, and general weakness | F | 77 | 16.6 | Positive | The patient went to hospital for supportive treatment and was discharged on the next day as there was no indication for hospitalization. Her main symptoms disappeared after 5days of NS therapy. COVID PCR was repeated after 12days of the having the therapy and showed a negative result. |
| 6 | 77-year-old patient with a history of Hyperlipidemia and Hypertension, Obesity and atrial fibrillation who presented with a shortness of breath, fever and dry cough | F | 77 | 80 | Negative | Significant improvement of her general health. NS was taken for 2 and half days. CRP after two and half days of treatment was 20.2 mg/L |
| 7 | Dry cough with general weakness and abdominal cramps and diarrhea | M | 23 | 66.9 | negative | Significant clinical improvement within 3days. CRP was controlled 3days it came down to 22.3 mg/L |
| 8 | Patient with fever without infect focus. | M | 38 | 38.1 | negative | Fever disappeared after 1day of treatment. CRP was performed after 3days of the therapy it came down to 10.5mg/L |
| 9 | Patient with a chief complains of Sore throat with fever. Her daughter was diagnosed with COVID-19 | F | 56 | Not measured | positive | Clinical symptoms rapidly improved after 3days |
| 10 | Patient presented to us with Fever, dry cough, running nose and general weakness | M | 39 | 73 | negative | Clinical symptoms rapidly improved after 3 days CRP was measured after 6days of treatment and was 5.7mg/L |
| 11 | Patient was diagnosed with COVID-19. She consulted us by telephone after 10days of the diagnosis as her general health was worsened with having pesky cough accompanied by chest pain, shortness of breath and fever. Treatment with NS was given at this time | F | 52 | Not measured | Positive | Clinical symptoms rapidly improved within 3 days. No shortness of breath, significantly reducing cough severity and frequency |
| 12 | Patient was hospitalized in the ICU due to severity of his COVID- 19 symptoms and has received supportive therapy. He consulted us by telephone after being discharged from hospital due to general weakness, dry cough accompanied with chest pain and shortness of breath | M | 50 | 24 | positive | Compositions comprising Nigella sativa extracts as a therapy was given at this time. The shortness of breath was significantly reduced within 3 days and patient developed a significant improvement of his general health. SARS-CoV-2 PCR was obtained on day 7of his NS Therapy and was negative. CRP was controlled and found within normal range. Chest x-ray was performed after 8 weeks of the therapy and showed normal findings |
| 13 | Patient presented to us with fever, general weakness, sweating | F | 68 | 127.2 | Negative | Significant improvement of her general health. Fever disappeared in the first 2days of the therapy. CRP dropped to 81.3 after two days of NS therapy |
| 14 | Patient presented to us with fever, dry cough, and general weakness | M | 51 | 84.5 | Negative | The symptoms were significantly reduced after two days. CRP was decreased to 40.5 during the second of the therapy. |
| 15 | Patient presented to us with fever, nasal congestion and dry cough | M | 48 | 45.6 | Negative | The symptoms were relieved completely after 4days. CRP dropped to 5.2 after 4 days of treatment |
| 16 | Fever with general weakness and headache | M | 27 | 114.4 | negative | The fever disappeared on the second day of treatment the patient gained his health back after two days. CRP was decreased to 36.2 after 3 days of the therapy |
| 17 | Patient presented with Dry cough, Fever, nasal congestion and sneezing | F | 56 | 58.5 | negative | According to patient the symptoms were significantly disappeared after 4days of treatment. CRP was not measured |
| 18 | Patient presented to us sweating with mild increased in her temperature. Flu symptoms were denied No infect focus was determined | F | 72 | 32.6 | Not performed | Symptoms were relieved after 3days. CRP was reduced to 14.6 on the 3^{rd} day of treatment |
| 19 | Patient presented to us with dry cough a mild shortness of breath | M | 72 | Not measured | positive | Clinical symptoms rapidly improved within 3 days. SARS-CoV-2 PCR was obtained on day 16 of his NS Therapy and was negative |
| 20 | Patient with productive cough, fever and malaise | M | 41 | 47.6 | negative | Clinical symptoms rapidly improved within 3 days-CRP reduced to 9.8 on day 3 of his NS Therapy |

The therapy which has been given to all patients consists of a combine capsule of NS and Vitamin E (ex. Alpinamed Schwarzkümmelöl, Schwarzkümmel plus capsules) with and without Iron oxide.

The therapy demonstrated a statistically significant time to improvement (mean time 3days) in clinical and respiratory symptoms and reduction in measured CRP.

RT-PCR tests are considered the gold standard for detecting many viruses, and quite a number of different tests are commericially available. Researchers at the Foundation for Innovative New Diagnostics, a nonprofit research center in Geneva, tested five COVID-19 RT-PCR tests and found that all five achieved 100% sensitivity on positive samples, and at least 96% specificity on negative samples in a laboratory setting. In practice, testing conditions and process are far from perfect, and accuracy suffers. It is still unclear what the real-world false positive rate is, but clinical sensitivity of RT-PCR tests ranges from 66% to 80%. That means nearly one in three infected people who are tested will receive false negative results.

## Claims

1. Composition comprising an extract of Nigella sativa for use in the treatment of a viral infection with SARS-CoV-2.

2. Composition according to claim 1, wherein the extract of Nigella sativa is further purified and comprises at least one active compound of Nigella sativa selected from the group consisting of thymoquinone, thymohydroquinone, p-cymene, α-hederin, carvacrol, anethol, 4-terpineol, thymol, alpha-pinene, limonene, nigellidine, and sesquiterpene longifolene.

3. Composition according to claim 1, wherein the extract of Nigella sativa is further purified and comprises at least one active compound of Nigella sativa, the at least one active compound being thymoquinone, optionally combined with further active compounds selected from the group consisting of thymohydroquinone, p-cymene, α-hederin, carvacrol, anethol, 4-terpineol, thymol, alpha-pinene, limonene, nigellidine, and sesquiterpene longifolene.

4. Composition according to claim 1, wherein the composition further comprises tocopherol for use in the treatment of a viral infection with SARS-CoV-2.

5. Composition according to claim 3, wherein the tocopherol is α-tocopherol.

6. Composition according to claim 1, wherein the composition further comprises an iron oxide compound for use in the treatment of a viral infection with SARS-CoV-2.

7. Kit of parts comprising the composition for use in the treatment of a viral infection with SARS-CoV-2 according to any of claims 1 to 6 and composition comprising ferrite nanoparticles.

8. Kit of parts according to claim 7, wherein the ferrite nanoparticles are selected from the group consisting of magnetite (Fe₃O₄) and maghemite (Fe₂O₃).
